# FASCICULE DE BREVET EUROPEEN

(11) **EP 1 989 541 B1**
(45) Date de publication et mention de la délivrance du brevet: **07.11.2012**
(21) Numéro de dépôt: 07710568.2
(22) Date de dépôt: 06.02.2007
(51) Int. Cl.: G01N 33/10, G01N 11/06

(54) **Procédé de mesure des qualités rhéologiques des pâtes fermentantes ou azymes à base de farine et dispositif pour sa mise en oeuvre**
Verfahren zur Messung der rheologischen Qualitäten von fermentiertem oder ungesäuertem Teig auf Mehlbasis, sowie Vorrichtung dafür
Method for measuring rheological qualities of fermented or unleavened flour-based dough and device therefor

(30) Priorité: 17.02.2006 BE 200600102
(43) Date de publication de la demande: 12.11.2008
(73) Titulaire: VANNESTE, Jacques, B-9051 Gent (BE)
(72) Inventeur: VANNESTE, Jacques, B-9051 Gent (BE)
(86) Numéro de dépôt international: PCT/BE2007/000015
(87) Numéro de publication internationale: WO 2007/093017

(56) Documents cités:
- FR-A1- 2 368 706
- GB-A- 428 174
- GB-A- 492 049
- SHARMA N ET AL: "Flow behavior of Wheat Flour-Water Dough Using a Capillary Rheometer I. Effect of Capillary Geometry" CEREAL CHEMISTRY, AACC INTERNATIONAL, ST PAUL, MN, US, vol. 70, no. 1, janvier 1993 (1993-01), pages 59-63, XP002409533 ISSN: 0009-0352

## Description

La présente invention concerne un procédé pour la mesure des qualités rhéologiques de pâtes, notamment des pâtes azymes ou fermentantes de farine de céréales.

On connaît du brevet GB 492 049 deux appareils utilisés en série décrits dans ce document, notamment un appareil d'extrusion qui extrude un échantillon de pâte sous forme d'un tube de très faible diamètre extérieur. La vitesse d'extrusion donne une évaluation du module d'élasticité. Le second appareil sert à étirer un échantillon de pâte ayant un module d'élasticité prédéterminé, pour mesurer l'extensibilité et la résistance à l'étirement. Ces deux mesures combinées y sont présentées comme ayant une valeur prédictive quant à la qualité boulangère de la farine de froment composant la pâte.

Deux appareils distincts sont donc requis : un appareil d'extrusion pour déterminer l'hydratation nécessaire afin d'obtenir une pâte ayant un module d'élasticité prédéterminé. Pour cela il faut pétrir trois pâtes avec des hydratations différentes. Ensuite, on pétrit une quatrième pâte ayant un module d'élasticité prédéterminé, pâte qui permet d'effectuer cinq étirements répétitifs. Une expérience complète avec les deux appareils employés en série dure environ deux heures. Ce procédé présente l'inconvénient d'être long et laborieux, et les nombreuses manipulations de la pâte durant les expériences mettent en cause la réproductibilité des résultats. (D.W Kent-Jones, 1967, Modern Cereal Chemistry, Food Trade Press London, p. 330-331-332).

Le principe même de l'extrusion par des orifices de faible diamètre est mis en question car la vitesse d'extrusion est influencée par les résistances variables qu'opposent les différentes pâtes à leur dégradation structurelle lors de leur passage par un tel orifice (H.G. Muller, 1968, S.C.I. Monograph n°27, §Discussion by P. Halton, p. 196). De plus, certaines pâtes peuvent coller à la paroi intérieure du cylindre d'où est extrudée la pâte. Ces deux facteurs influencent la vitesse d'extrusion et faussent donc la mesure même dudit module d'élasticité qui sert de base à la mesure des caractéristiques rhéologiques obtenues avec le second appareil.

La présente invention a pour but de remédier aux inconvénients indiqués ci-dessus. A cette fin il est proposé suivant l'invention un procédé tel que défini dans la revendication principale. Le procédé d'extrusion selon l'invention permet de mesurer simultanément un indice de la consistance de la pâte et un indice de sa recouvrance de déformation élastique, dans lequel ces mesures ne sont pas influencées par le degré de collant de la pâte, ni par sa dégradation structurelle.

Les buts sont atteints par l'invention telle qu'elle est définie dans les revendications, et au premier chef dans la revendication principale, notamment en utilisant un appareil d'extrusion, qui produit, par une extrusion verticale et ascendante, des pâtons sphéroïdaux à aplatissement variable et de volume constant, à partir de pâtes ayant de préférence des consistances courantes en panification.

Ainsi les pâtons extrudés sont des sphéroïdes. Cette forme résulte d'une sélection appropriée des diamètres de l'orifice et de la cavité de l'appareils d'extrusion et du fait que la recouvrance de déformation élastique de la pâte, après son passage par l'orifice d'extrusion, est biaxiale et que les caractéristiques rhéologiques de la pâte ne sont pas dégradées par ce mode d'extrusion. Suivant un autre mode de réalisation préféré de l'invention, les surfaces des installations en contact avec la pâte sont revêtues d'un matériau anti-adhésif et/ou lubrifiées pour neutraliser l'effet de collage de certaines pâtes.

C'est uniquement sous ces conditions que la vitesse d'extrusion est un indice valable de la consistance des pâtes.

Grâce au procédé suivant l'invention, la durée d'une expérience est réduite vu que les mesures se font simultanément avec un seul appareil et qu'il ne faut qu'un seul temps de repos et un seul pétrissage par expérience qui comprend, par exemple, trois essais répétitifs.

Les autres avantages obtenus grâce à la présente invention consistent essentiellement en ce que le procédé de mesure est simple, rapide et efficace.

En outre, la hauteur "H" du pâton extrudé est un paramètre qui a une très forte valeur prédictive quant à la qualité boulangère de la farine, composante de cette pâte à hydratation égale, tout en étant un indice de la recouvrance de déformation élastique.

Un autre avantage significatif obtenu réside dans le fait que la durée d'extrusion, qui est un indice de la consistance de la pâte pour une hydratation donnée de la farine composante de cette pâte, a une très forte valeur prédictive concernant la consistance des pâtes que l'on obtiendra en panification, avec la même hydratation et même farine.

Par ailleurs, les mesures des indices de la consistance et de la recouvrance de déformation élastique obtenues suivant l'invention sont cohérentes vu qu'elles sont effectuées simultanément sur le même échantillon de pâte.

Enfin une expérience, dure environ 20 minutes pour trois essais répétitifs qui la composent, essais qui n'exigent qu'une manipulation simple de la pâte.

La présente invention concerne également une installation pour la mise en oeuvre du procédé suivant l'invention défini ci-dessus. Ainsi, l'installation de mesure suivant l'invention est robuste et économique.

De plus, le procédé combiné à l'installation suivant l'invention permet d'étudier des pâtes azymes et des pâtes fermentantes, ainsi que les effets de l'acide ascorbique et autres additifs sur ces pâtes.

Enfin l'installation et le procédé sont aussi bien applicables à un laboratoire de recherche qu'à un laboratoire de contrôle de qualité en meunerie et aussi en boulangerie, d'autant plus que l'on peut tester des pâtes fermentantes prélevées en cours de panification.

L'invention est décrite ci-après de façon plus détaillée à l'aide d'un exemple non limitatif avec référence aux dessins annexés dans lesquels:
La figure 1 représente une coupe verticale médiane de l'installation de mesure conformément à l'invention.
La figure 2 représente une coupe verticale médiane d'un chargeur conformément à une caractéristique additionnelle de l'invention.

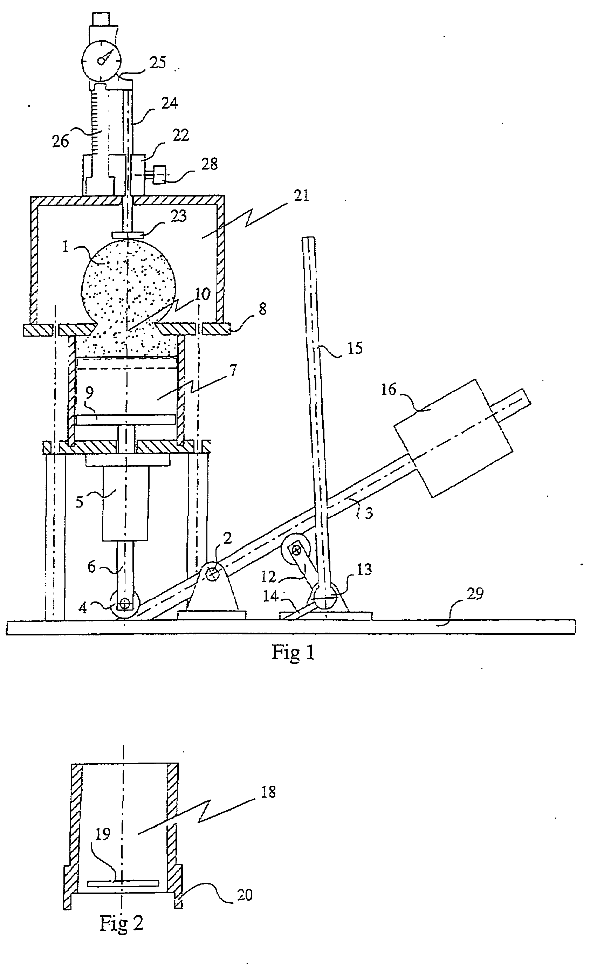

La figure 1 montre l'installation d'extrusion, comprenant essentiellement une cavité 7 par exemple de forme cylindrique prévue pour recevoir un échantillon de pâte. Dans cette cavité 7 de préférence thermostatée peut coulisser un piston 9 guidé par une tige 6. La cavité peut être fermée par un couvercle 8 dans lequel est aménagée une filière d'extrusion 10 qui peut être obturée par un poids non représenté sur les dessins.

Dans le bord supérieur de la paroi formant la cavité 7 sont aménagées de fines encoches, non représentées sur les dessins, qui permettent à l'air et à l'huile de s'échapper hors de la cavité 7 par dessous le couvercle 8 lors de la montée du piston 9.

La cavité 7 est équipée d'une sonde de thermomètre, non représentée sur les dessins, qui est placée de telle façon qu'elle pénètre l'échantillon de pâte.

Conformément à la présente invention, un levier 3 pouvant basculer sur un pivot 2 actionne le piston 9. Les différentes positions du levier 3 sont commandées par un mécanisme qui se compose d'un axe 13 pouvant pivoter dans deux paliers représentés que schématiquement sur les dessins. Sur cet axe 13 sont montés un levier 12 équipé d'un galet, un arrêt 14 qui peut venir s'appuyer sur une plaque de support 29 et un levier de commande 15. Le bras long du levier 3 muni d'un contrepoids 16 peut être manoeuvré par le levier 12, tandis que le bras court du levier actionne le piston 9. La tige 6 est guidée par une douille 5 munie de roulements pour mouvements axiaux à son extrémité elle est équipée d'un galet 4 à gorge qui peut se déplacer longitudinalement sur le levier 3 qui est sensiblement cylindrique. Ce mécanisme permet de bloquer le piston 9 dans sa position basse quand l'arrêt 14 repose sur la plaque de support 29 d'une part, et de laisser monter le piston 9, d'autre part, de telle façon que l'échantillon de pâte contenu dans la cavité 7 soit comprimé progressivement et sans à coups, l'orifice 10 étant obturé par le poids, non représenté sur les dessins, et qu'ensuite elle soit extrudée sous pression constante et sans saccades par l'orifice d'extrusion 10, quand le levier 3 peut basculer librement. Un commutateur électrique non représenté sur les dessins est actionné quand le poids obturant l'orifice d'extrusion 10, est soulevé et enclenche le chronomètre.

Dans la figure 1 le piston 9 est aussi représenté en pointillés dans sa position supérieure, dans laquelle une partie de l'échantillon de pâte est déjà extrudée sous la forme d'un pâton 1 sphéroïdal ayant un aplatissement variable. Dès que le piston 9 atteint sa position supérieure, le levier 3 actionne un commutateur électrique non représentés sur les dessins qui arrête le chronomètre.

La figure 2 montre un chargeur qui est destiné à recevoir et laisser reposer un échantillon de pâte et qui comprend une cavité 18 ayant une conicité dont le diamètre maximal est agencé à proximité d'un tiroir 19. Le chargeur est muni d'un rebord 20 pouvant s'emboîter sur le bord supérieur de la cavité 7 de l'installation d'extrusion montré sur la figure 1. Le tiroir est constitué d'une plaque sensiblement plane et rectangulaire non représentée sur les dessins, qui est moins large que le diamètre maximal de la cavité 18 et qui peut coulisser dans deux fentes 19 aménagées en vis-à-vis dans la paroi de la cavité 18.

Un dispositif de mesure illustré sur la figure 1 comprend une enceinte 21 de préférence cylindrique et transparente, par exemple du plexiglass®, dont le couvercle est pourvu en son centre d'un dispositif qui se compose d'un coussinet 22 et dans lequel peut coulisser axialement et verticalement la tige 24 équipée à son extrémité inférieure d'un disque palpeur 23, la tige 24 pouvant être bloquée dans une position quelconque par un écrou 28 dont est muni le coussinet 22. Sur le coussinet 22 est aussi fixé une règle graduée 26 munie d'un coulisseau 25 avec un vernier gradué et un bec, lequel peut être descendu sur l'extrémité supérieure de la tige 24.

Ce dispositif de mesure amovible est agencé concentriquement à l'orifice d'extrusion 10 sur le couvercle 8 qui est agencé dans un plan horizontal de façon que l'axe de la tige 24 du disque palpeur 23 coïncide avec celui de la cavité 7.

Les surfaces du dispositif de mesure et de l'installation auxiliaire en contact avec la pâte sont avantageusement revêtues d'une matière anti-adhésive et/ou antifriction et/ou lubrifiées.

Un autre dispositif de mesure, non représenté sur les dessins, est constitué d'un mesureur de distance par rayon laser qui est par exemple monté sur le dessus de l'enceinte 21.

Un exemple de mode d'utilisation du dispositif de mesure pour la mise en oeuvre du procédé de mesure est décrit ci-après. Le piston 9 se trouve dans sa position inférieure, la face supérieure du piston 9 et l'intérieur de la cavité 7 sont huilés ainsi que la paroi intérieure de la cavité 18 et le tiroir 19 des chargeurs montrés sur la figure 2. On pétrit une pâte, par exemple, de farine de froment, d'eau de sel et de levure, et ceci dans des conditions déterminées. Cette pâte est alors divisée en par exemple trois échantillons identiques qui sont façonnés en boules de façon constante par une bouleuse mécanique de laboratoire. Ensuite on loge les échantillons identiques de pâte façonnée en boules dans les chargeurs. Après un temps de repos dans une enceinte de préférence thermosthatée non représentée sur les dessins, le chargeur précité est emboîté sur la cavité 7, le piston 9 se trouvant alors dans sa position inférieure, on ouvre le tiroir et l'échantillon de pâte tombe dans la cavité 7.

Ensuite, on enlève le chargeur et on fixe le couvercle 8 et on obture l'orifice d'extrusion 10 par un poids muni d'un commutateur électrique non représenté sur les dessins. La face du poids obturant la filière d'extrusion 10 est huilée. En manoeuvrant le levier de commande 15, on laisse remonter lentement le piston 9 jusqu'au point ou la pâte est soumise à l'entière pression exercée par le poids 16. Ensuite, le levier de commande 15 est placé dans sa position inférieure de telle façon que le galet 12 du levier 13 ne soutient plus le levier 3 qui peut ainsi atteindre librement sa position inférieure, de sorte que l'échantillon de pâte est comprimé pour une durée déterminée. Ensuite, la pâte étant toujours sous pression, on soulève vivement le poids et le commutateur fixé sur le poids enclenche un chronomètre, et la pâte contenue dans la cavité 7 est extrudée par l'orifice 10. Lorsque le piston 9 atteint sa position supérieure, l'extrusion est terminée et le chronomètre est arrêté par un commutateur, non représenté sur les dessins, actionné par le levier 3 en fin de course et la durée d'extrusion est enregistrée. Ensuite, le dispositif de mesure précité est mis en place sur le couvercle 8, le palpeur 23 se trouvant dans sa position supérieure, de telle façon que l'axe de la tige 24 coïncide sensiblement avec l'axe 1 de la cavité 7. Ensuite la tige 24 du palpeur 23 est libérée en desserrant l'écrou 28, le palpeur 23 descend et se pose sur le pâton 1. Ensuite, on bloque la tige 24 du palpeur 23 à l'aide de l'écrou 28 et on lit la hauteur "H" du pâton en déterminant la position du coulisseau à vernier 25 sur l'échelle graduée 26.

La hauteur "H" est mesurée à partir de la base du pâton 1 se situant dans le plan de la sortie de l'orifice d'extrusion 10. Plusieurs mesures successives, de la hauteur "H" et de la durée d'extrusion peuvent être effectuées sur une série de par exemple trois échantillons identiques. La moyenne des mesures améliore la répétitivité et la reproductibilité des résultats.

On relève la température de l'échantillon de pâte contenu dans la cavité 7. Si cette température diffère sensiblement d'une éventuelle température de référence prédéterminée pour les échantillons de pâte, on corrige l'indice de consistance mesuré en fonction de la différence entre la température prédéterminée et la température relevée.

Après avoir enlevé le pâton 1 et la pâte résiduelle hors de la cavité 7, l'installation est prête pour l'essai suivant. Vu que les surfaces en contact avec la pâte sont revêtues d'un matériau anti-adhésif et/ou huilées, il n'y a pas de problèmes de collage aux surfaces.

La présente invention n'est pas limitée aux seules formes de réalisation qui ont été décrites ci-dessus mais elle en embrasse toutes les variantes de réalisation, comme définies dans les revendications suivantes.

## Revendications

1. Procédé de mesure simultanée des qualités rhéologiques des pâtes fermentantes ou azymes à base de farine, à partir d'un pâton (1), **caractérisé en ce que** le pâton est obtenu par une extrusion verticale et ascendante d'un échantillon de pâte de façon à lui conférer une forme sphéroïdale à aplatissement variable, et de volume sensiblement constant et **en ce que** les mesures d'un indice de la consistance et d'un indice de la recouvrance de déformation élastique sont effectuées simultanément sur le même pâton (1).

2. Procédé de mesure selon la revendication 1, **caractérisé en ce que** l'échantillon de pâte est mis sous pression (P), et **en ce que** la mise sous pression de l'échantillon de pâte, avant extrusion, est effectuée d'une façon progressive dans laquelle la pression (P) est telle qu'elle n'altère pas les qualités rhéologiques du pâton (1) lors de son extrusion, et qu'ensuite l'extrusion se fait sous pression sensiblement constante et sans à-coups.

3. Procédé de mesure selon la revendication 1 ou 2, **caractérisé en ce que** le pâton (1) est maintenu en place après son extrusion de façon à reposer sur la pâte contenue dans l'orifice d'extrusion (10), de façon à être disposé convenablement pour être mesuré.

4. Procédé de mesure selon l'une des revendications précédentes 1 et 2, **caractérisé en ce que** l'on mesure la durée d'extrusion du pâton (1) cette durée étant un indice de la consistance du pâton (1).

5. Procédé de mesure selon l'une quelconque des revendications précédentes 2 à 4, **caractérisé en ce que** l'on mesure, après l'extrusion, la hauteur (H) du pâton (1) constituant un indice de la recouvrance de déformation élastique du pâton (1), par des moyens de mesure.

6. Procédé de mesure selon la revendication 5, **caractérisé en ce qu'**on mesure la hauteur (H) du pâton (1) qui est proportionnelle à son diamètre.

7. Procédé de mesure selon la revendication précédente, **caractérisé en ce que** les mesures des indices de consistance et de la recouvrance de la déformation élastique sont répétées sur une série d'échantillons identiques de pâte, conditionnés et reposés de façon appropriée avant leur extrusion.

8. Procédé de mesure selon l'une des revendications 5 à 7, **caractérisé en ce que** l'on lubrifie les surfaces des dispositifs de mesure et d'un dispositif additionel amenées à être en contact avec l'échantillon de pâte.

9. Procédé de mesure selon la revendication 2, **caractérisé en ce que** la pression "P" est directement obtenue par gravité.

10. Dispositif de mesure pour la mise en oeuvre du procédé de mesure selon la revendication 1, **caractérisé en ce qu'**il comprend des moyens pour former le pâton (1) par extrusion et **en ce que** les moyens pour former le pâton (1) sont disposés de façon telle que l'extrusion du pâton (1) est effectuée de façon verticale et ascendante.

11. Dispositif de mesure selon la revendication 10 pour la mise en oeuvre du procédé de mesure selon la revendication 2, **caractérisé en ce qu'**un bras long d'un levier (3) est soulevé ou libéré par un levier court à galet (12) monté sur un arbre (13) qui est équipé d'un levier long de commande (15) et d'un arrêt (14) pour maintenir un piston (9) dans sa position basse.

12. Dispositif de mesure selon la revendication 10 pour la mise en oeuvre du procédé de mesure selon la revendication 2, **caractérisé en ce que** les moyens pour mettre et maintenir sous pression l'échantillon de pâte contenu dans une première cavité (7) et ensuite de régler l'extrusion du pâton (1) se composent d'un levier (3) muni d'un contrepoids (16), le levier (3) s'appuyant sur un pivot (2) et actionnant un piston (9) par l'intermédiaire d'une tige (6) sur laquelle il est fixé.

13. Dispositif de mesure selon la revendication 12, **caractérisé en ce que** la translation de la tige (6) est guidée par une douille (5) munie de roulements à billes pour mouvements linéaires axiaux.

14. Dispositif de mesure selon la revendication 12, **caractérisé en ce que** l'extrémité de la tige (6) est munie d'un galet à gorge (4) pouvant rouler longitudinalement sur le levier (3).

15. Dispositif selon la revendication 12 pour la mise en oeuvre du procédé selon l'une des revendications 1 à 8, **caractérisé en ce qu'**il comprend un dispositif additionnel comprenant un chargeur présentant une seconde cavité (18) dans laquelle est logé l'échantillon de pâte, ladite seconde cavité présentant une forme sensiblement tronconique dont la base est adjacente à deux tiroirs, **en ce que** le diamètre maximal de ladite seconde cavité (18) est inférieur au diamètre de la première cavité (7), **en ce que** les deux tiroirs précités sont constitués chacun de plaques sensiblement planes moins larges que le diamètre maximal de la dite seconde cavité (18), et **en ce que** les tiroirs précités sont agencés à coulissement en sens mutuellement opposé, chacun dans l'une des deux fentes (19) aménagées en vis-à-vis dans la paroi de ladite seconde cavité (18).

## Claims

1. A method for simultaneously measuring the rheological properties of fermenting or unleavened flour based doughs using a dough piece (1), **characterized in that** the dough piece (1) is is obtained by a vertical and upward extrusion of a dough sample to conferto the dough piece a spheroidal shape with variable flattening and a constant volume, and **characterized in that** an index of dough consistency and an index of the recovery of elastic deformation are simultaneously measured on the same dough piece (1).

2. The measuring method according to claim 1, **characterized in that** the dough sample is put under pressure (P), wherein the pressurization of the dough sample prior to extrusion is carried out in a progressive manner and wherein the pressure (P) is such that it does not alter the rheological qualities ofthe dough piece (1) during its extrusion, and that subsequentley the extrusion is performed under substantially constant pressure and in a smooth manner.

3. The measuring method according to claims 1 or 2, **characterized in that** the dough piece (1) is held in place after its extrusion so as to rest on the dough contained in the extrusion orifice (10), so as to be disposed properly for measurement.

4. The measuring method according to one ofthe preceding claims 1 and 2, **characterized in that** the duration of extrusion of the dough piece (1) is measured wherein said duration is an index of the consistency ofthe dough piece (1).

5. The measuring method according to any one of preceding claims 2 to 4, **characterized in that** the height (H) of the dough piece (1), after extrusion, is measured, wherein this height (H) is an index ofthe recovery of the elastic deformation of the dough piece (1).

6. The measuring method according to claim 5, **characterized in that** the height (H) of the dough piece (1) is measured which is proportional to its diameter.

7. The measuring method according to the preceding claim, **characterized in that** the measurements of the consistency index and the index of the recovery of the elastic deformation are repeated on a series of identical dough samples, appropriately conditioned and rested prior to extrusion.

8. The measuring method according to one ofthe claims 5 to 7, **characterized in that** the surfaces of the measurement device and an additional device that are in contact with the dough sample are lubricated.

9. The measuring method according to claim 2, **characterized in that** the pressure "P" is directly obtained by gravity.

10. Measurement device for the implementation of the measuring method according to claim 1, **characterized in that** said device comprises means for forming the dough piece (1) by extrusion and wherein said means for forming the dough piece (1) are arranged so that the extrusion of the dough piece (1) is conducted vertically and upward.

11. The measurement device according to claim 10 for implementing the measuring method according to claim 2, **characterized in that** a long arm of a lever (3) is raised or released by a short roller lever (12) mounted on a shaft (13) which is equipped with a long control lever (15) and a stop (14) for holding a piston (9) in its lower position.

12. The measurement device according to claim 10 for implementing the method according to claim 2, **characterized in that** the means for putting and keeping the dough sample contained in a first cavity (7) under pressure and for subsequently controlling the extrusion ofthe dough piece (1) comprise a lever (3) equipped with a counterweight (16), wherein the lever (3) is based on a pivot (2) and activates a piston (9) through a rod (6) to which it is attached.

13. The measurement device according to claim 10, **characterized in that** the movement of the rod (6) is is guided by a sleeve (5) provided with ball bearings for axial linear movement.

14. The measurement device according to claim 12, **characterized in that** the end of the rod (6) is provided with a grooved roller (4) that can run longitudinally on the lever (3).

15. The measurement device according to claim 12 for implementing the measuring method according to claims 1 to 8, **characterized in that** said device comprises an additional device comprising a charger having a second cavity (18) in which the dough sample is placed, wherein said second cavity has a substantially truncated cone shape whose base is adjacent to two drawers, wherein the maximum diameter of said second cavity (18) is smaller than the diameter of the first cavity (7), wherein each of said two drawers consists of a substantially planar plate less wide than the maximum diameter of said second cavity (18) and wherein said drawers are arranged to slide in mutually opposite directions, each drawer in one of the two slots (19) located in opposite sides in the wall of said second cavity (18).

## Patentansprüche

1. Verfahren für das zeitgleiche Messen der Rheologischen Eigenschaften fermentierende oder hefefreie Mehlteige anhand eines Teigstücks (1), das sich **dadurch kennzeichnet, dass** das Teigstück über eine senkrechte und aufsteigende Extrusion eines Teigmusters hergestellt wird, damit es kugelförmig ist sowie eine variable Abflachung und ein konstantes Volumen hat, und dass der Index für die Konsistenz sowie der Index für die Rückverformung elastischer Verformungen zeitgleich mit demselben Teigstück gemessen werden (1).

2. Messverfahren gemäß Anforderung Nr. 1, das sich **dadurch kennzeichnet, dass** auf dem Teigmuster Druck (P) ausgeübt wird und dass der Druckaufbau, der vor der Extrusion stattfindet, so schrittweise stattfindet, dass der Druck (P) die Rheologischeneigenschaften des Teigstück(1)s während der Extrusion nicht ändert, und dass die Extrusion mit einem konstanten Druck und ohne Schläge stattfindet.

3. Messverfahren gemäß Anforderung 1 oder 2, das sich **dadurch kennzeichnet, dass** das Teigstück (1) nach der Extrusion an Ort und Stelle gehalten wird, so dass es auf dem Teig liegt, der sich in der Öffnung für die Extrusion (10) befindet, und so dass es korrekt angeordnet ist im Hinblick auf die Messung.

4. Messverfahren gemäß einer der Anforderungen 1 und 2, das sich **dadurch kennzeichnet, dass** die Dauer der Extrusion des Teigstücks (1) gemessen wird, wobei die Dauer ein Index für die Konsistenz des Teigstücks (1) darstellt.

5. Messverfahren gemäß einer der Anforderungen 2 bis 4, das sich **dadurch kennzeichnet, dass** die Höhe (H) des Teigstückes (1) nach der Extrusion mit Hilfe von Messgeräten gemessen wird, wobei die Höhe ein Index für die Rückverformung der elastischen Verformung des Teigstücks (1) darstellt.

6. Messverfahren gemäß der Anforderung 5, das sich **dadurch kennzeichnet, dass** die Höhe (H) des Teigstücks (1) gemessen wird, welche sich proportional zu seinem Durchmesser verhält.

7. Messverfahren gemäß der vorigen Anforderung, das sich **dadurch kennzeichnet, dass** die Messungen des Indexes für die Konsistenz und des Indexes für die Rückverformung der elastischen Verformung mit einer Reihe identischer Teigmuster wiederholt werden, die vor ihrer Extrusion ordnungsgemäß verpackt wurden und geruht haben.

8. Messverfahren gemäß einer der Anforderungen 5 bis 7, das sich **dadurch kennzeichnet, dass** die Flächen der Messgeräte und eines Zusatzgeräts, die mit dem Teigmuster in Berührung kommen werden, geschmiert werden.

9. Messverfahren gemäß der Anforderung 2, das sich **dadurch kennzeichnet, dass** der Druck "P" direkt über die Schwerekraft erzielt wird.

10. Messgerät für die Durchführung des Messverfahrens gemäß Anforderung 1, das sich **dadurch kennzeichnet, dass** es Mittel umfasst, um das Teigstück (1) mittels Extrusion zu formen, und dass die Mittel, um das Teigstück (1) zu formen, so angeordnet sind, dass die Extrusion des Teigstückes (1) senkrecht und aufsteigend stattfindet.

11. Messgerät gemäß Anforderung 10 im Hinblick auf die Durchführung des Messverfahrens gemäß Anforderung Nr. 2, das sich **dadurch kennzeichnet, dass** ein langer Hebelarm (3) angehoben oder befreit wird von einem kurzem Anfahrrollenhebel (12), der sich auf einer Welle (13) befindet, die mit einem langen Steuerhebel (15) und einem Anschlag (14) ausgestattet ist, damit ein Kolben (9) in seiner unteren Position bleibt.

12. Messgerät gemäß Anforderung 10 im Hinblick auf die Durchführung des Messverfahrens gemäß Anforderung Nr. 2, das sich **dadurch kennzeichnet, dass** die Instrumente, die das im ersten Hohlraum (7) befindliche Teigmuster unter Druck setzen und halten müssen und die anschließend die Extrusion des Teigstücks (1) regeln müssen, aus einem Hebel (3) mit einem Gegengewicht 16) bestehen, wobei der Hebel (3) auf einen Zapfen (2) stützt und über eine Stange (6), auf der er befestigt ist, einen Kolben (9) in Bewegung setzt.

13. Messgerät gemäß Anforderung 10, das sich **dadurch kennzeichnet, dass** die Bewegung der Stange (6) von einer Buchse (5), die mit Kugellagern für lineare axiale Bewegungen ausgestattet ist, geführt wird.

14. Messgerät gemäß Anforderung 12, das sich **dadurch kennzeichnet, dass** das Ende der Stange (6) mit einer genuteten Laufrolle (4) versehen ist, die in der Längsrichtung auf dem Hebel (3) laufen kann.

15. Messgerät gemäß Anforderung 12 im Hinblick auf die Durchführung des Messverfahrens gemäß einer der Anforderungen 1 bis 8, das sich **dadurch kennzeichnet, dass** es ein Zusatzgerät mit einem Lader und einem zweiten Hohlraum (18) umfasst, in dem sich das Teigmuster befindet, wobei der zweite Hohlraum eine kegelstumpfe Form aufweist, dass der Höchstdurchmesser des obenerwähnten zweiten Hohlraums (18) kleiner ist als der Höchstdurchmesser des ersten Hohlraums (7), dass die beiden obenerwähnten Schubläden aus im Wesentlichen flachen Platten bestehen, die weniger breit sind als der Höchstdurchmesser des zweiten Hohlraums (18) und dass die obenerwähnten Schubläden jeweils hin und her geschoben werden und zwarjede in einer der beiden Aufnahmen (19), die direkt gegenüber in die Seitenwand des zweiten Hohlraums (18) eingearbeitet sind.
